(19) Europäisches Patentamt
European Patent Office
Office européen des brevets

(11) Publication number: **0 031 653**
**A1**

(12) **EUROPEAN PATENT APPLICATION**

(21) Application number: **80304407.2**

(22) Date of filing: **05.12.80**

(51) Int. Cl.³: **C 07 C 101/04,** C 07 D 209/20, C 07 D 213/55, C 07 D 317/58, C 07 C 149/24, A 61 K 31/195, A 61 K 31/445, A 61 K 31/405, A 61 K 31/34

(30) Priority: **19.12.79 GB 7943794**

(43) Date of publication of application: **08.07.81 Bulletin 81/27**

(84) Designated Contracting States: **AT BE CH DE FR GB IT LI NL SE**

(71) Applicant: **BEECHAM GROUP LIMITED, Beecham House Great West Road, Brentford, Middlesex (GB)**

(72) Inventor: **Ferres, Harry, 20 Garlichill Road, Epsom Surrey (GB)**

(74) Representative: **Russell, Brian John et al, European Patent Attorney Beecham Pharmaceuticals Yew Tree Bottom Road, Epsom Surrey, KT18 5XQ (GB)**

(54) **N-Substituted alpha-amino acids, processes for making them and pharmaceutical compositions containing them.**

(57) Compounds of the formula (II):

$$R^2\text{-}CH_2\text{-}NH\text{-}\underset{\alpha}{\overset{\overset{\displaystyle R^1}{|}}{C}H}\text{-}CO_2H \qquad \text{(II)}$$

wherein $R^1$ represents an optionally substituted aryl, aralkyl, aromatic heterocyclic or aromatic heterocyclylalkyl group, and $R^2$ represents a phenyl group optionally substituted with $C_{1-6}$ alkyl, halogen, hydroxy, trifluoromethyl or methylenedioxy, with the proviso that when $R^1$ is benzyl, $R^2$ is phenyl, 4-chlorophenyl, 2-hydroxyphenyl, $\alpha$-$C_{10}H_7$– or $\beta$-$C_{10}H_7$–, have been found to have useful hyperalphaliproproteinaemic and/or hypobetalipoproteinaemic properties.

These compounds are prepared by methods usual for analogous compounds.

EP 0 031 653 A1

COMPLETE DOCUMENT

# N-Substituted α-Amino Acids, Processes For Making Them And Pharmaceutical Compositions Containing Them

This invention relates to a class of N-substituted α-amino acids which are potentially useful in the treatment of atherosclerosis.

The invention also relates to processes for the preparation of such compounds and pharmaceutical compositions comprising them.

N-Substituted α-amino acids, in particular N-benzyl-α-amino acids, have been used extensively as protected derivatives for use in peptide synthesis; however, relatively few simple amino acid derivatives have been investigated in respect of bioactivity.

The N-substituted-DL-amino acids of formula (I) are known from J. Pharm. Soc. Japan, 1978, 98 (7) 869-879.

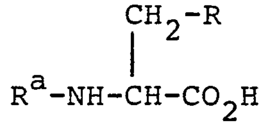

(I)

wherein R is phenyl and $R^a$ is benzyl, 4-chlorobenzyl, 2-hydroxybenzyl, α-$C_{10}H_7CH_2$-, β-$C_{10}H_7CH_2$- and cyclo $C_6H_{11}$-; and wherein R is hydrogen and $R^a$ is benzyl and

4-chlorobenzyl.

Biological tests, including antibacterial activity and the action on central nervous, circulation and muscular systems were carried out; however, the publication discloses that none of the compounds showed much activity.

It has now been found that a class of N-substituted amino acids, many of which are novel compounds, promote a more favourable composition of lipoproteins in the bloodstream, i.e. they are either especially hyperalphalipoproteinaemic [i.e. raise the blood levels of high-density lipoproteins (HDL)], with some hypobetalipoproteinaemic behaviour [i.e. lower blood levels of the low-density lipoproteins (LDL) and/or the very low-density lipoproteins (VLDL)] or exert both effects.

Accordingly the present invention provides an α-amino acid derivative of formula (II) or a pharmaceutically acceptable salt, ester or bioprecursor thereof:

$$R^2\text{-}CH_2\text{-}NH\text{-}\underset{\alpha}{\overset{R^1}{\overset{|}{C}H}}\text{-}CO_2H \qquad (II)$$

wherein $R^1$ represents an optionally substituted aryl, aralkyl, aromatic heterocyclic or aromatic

heterocyclylalkyl group, and $R^2$ represents a phenyl group optionally substituted with $C_{1-6}$ alkyl, halogen, hydroxy, trifluoromethyl or methylenedioxy, with the proviso that when $R^1$ is benzyl, $R^2$ is phenyl, 4-chlorophenyl, 2-hydroxyphenyl, $\alpha$-$C_{10}H_7$- or $\beta$-$C_{10}H_7$-, the compound of formula (II) is not in the DL-form.

Preferably $R^1$ represents phenyl or benzyl optionally substituted with halogen, lower alkyl or hydroxyl, or a 5 or 6 membered heterocyclic group containing one or more nitrogen, oxygen or sulphur atoms and optionally substituted with lower alkylene, or a bicyclic aromatic heterocyclic group containing one or more nitrogen, oxygen or sulphur atoms.

Preferred $R^1$ groups are indolylmethyl, or 2-picolyl.

A preferred $R^2$ group is the 4-halophenyl group, particularly 4-chlorophenyl.

Compounds of formula (II) contain at least one assymetric carbon atom (marked by the $\alpha$) and hence may exist in two optically active forms, the D-form and the L-form or as an optically inactive racemate. Included within the scope of this invention, therefore, are the substantially pure optical isomers, the racemate and mixtures thereof.

Preferably the compounds (II) are in the substantially pure L-form.

The term "pharmaceutically acceptable bioprecursor" used above requires some explanation. It is, of course, common practice in pharmaceutical chemistry to overcome some undesirable physical or chemical property of drug

by converting the drug into a chemical derivative which does not suffer from that undesirable property, but which, upon administration to an animal or human being, is converted back to the parent drug. For example, if the drug is not well absorbed when given to the animal or patient, by the oral route, it may be possible to convert the drug into a chemical derivative which is well absorbed and which in the serum or tissues is reconverted to the parent drug. For example, if the drug is unstable in solution, it may be possible to prepare a chemical derivative of the drug which is stable and may be administered in solution, but which is reconverted in the body to give the parent drug. The pharmaceutical chemist is well aware of the possibility of overcoming intrinsic deficiencies in a drug by chemical modifications which are only temporary and are reversible upon administration to the animal or patient.

For the purpose of this specification the term "pharmaceutically acceptable bioprecursor" of a drug means a compound having structural formula different from the drug which nonetheless, upon administration to an animal or human being, is converted in the patient's body to the drug.

Suitable pharmaceutically acceptable bioprecursors include *in vivo* hydrolysable ester groups.

Examples of groups which hydrolyse readily in the body to produce the parent acid include acyloxyalkyl groups such as acetoxymethyl, pivaloyloxymethyl, α-acetoxyethyl, α-acetoxybenzyl and α-pivaloyloxyethyl groups; alkoxycarbonyloxyalkyl groups, such as ethoxycarbonyloxymethyl and α-ethoxycarbonyloxyethyl; dialkylaminoalkyl groups such as dimethylaminomethyl,

dimethylaminoethyl, diethylaminomethyl or diethylaminoethyl; and lactone groups such as phthalidyl.

Suitable carboxylic acid salts of compound (II) include alkali metal salts such as lithium, sodium and potassium, other metal salts such as barium, calcium, aluminium or ammonium or substituted ammonium salts.

Suitable acid addition salts of compound (II) included inorganic salts such as the sulphate, nitrate, phosphate and borate, hydrohalides such as the hydrochloride, hydrobromide and hydroiodide, and organic acid addition salts such as acetate, oxalate, tartrate, maleate, citrate, succinate, benzoate, ascorbate, methanesulphonate and p-toluenesulphonate.

Preferred esters of the compounds of formula (II) are lower alkyl esters, particularly the methyl ester.

A sub-group of compounds within the scope of this invention comprises compounds of formula (III) or a pharmaceutically acceptable salt, ester or bioprecursor thereof:

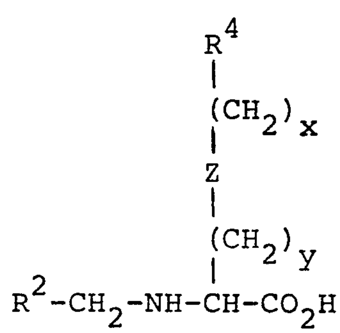

(III)

wherein $R^2$ is as defined with respect to formula (II), $R^4$ is phenyl optionally substituted with halogen or hydroxy, a 5 or 6 membered heterocyclic group containing one or more nitrogen, oxygen or sulphur atoms, or a bicyclic aromatic heterocyclic group containing one or

more nitrogen, oxygen or sulphur atoms, x and y are each an integer of from 1 to 4, and Z is an oxygen or sulphur atom or is a bond.

Preferably $R^4$ is phenyl, 4-hydroxyphenyl, 4-chlorophenyl, indolyl or pyridyl. Preferably, x and y are each 1 or 2.

The compounds of this invention may be prepared by reacting an amino compound of formula (IV):

$$\begin{array}{c} R^1 \\ | \\ H_2N\text{-}\underset{\alpha}{CH}\text{-}CO_2R^x \end{array} \qquad (IV)$$

wherein $R^1$ is as defined with respect to formula (II) and any reactive groups therein may be protected, and $R^x$ is hydrogen or a carboxy blocking group, with a carbonyl compound of either formula (V).

$$R^2 - CHO \qquad (V)$$

wherein $R^2$ is as defined with respect to formula (II) and any reactive groups may be protected, followed by reduction of the resulting Schiff's base with a suitable hydridic reducing agent, or hydrogen in the presence of a catalyst; and thereafter, if necessary, carrying out one or more of the following steps:

i) removing any carboxyl-blocking group $R^x$;

ii) removing any protecting groups on $R^1$ or $R^2$;

iii) converting the product into a pharmaceutically acceptable salt, ester or a pharmaceutically acceptable bioprecursor.

Suitable carboxyl-blocking derivatives for the group $-CO_2R^x$ in formula (II) include salts and ester derivatives of the carboxylic acid. The derivative is preferably one which may readily be cleaved at a later stage of the reaction. Suitable salts include tertiary amine salts, such as those with tri-lower alkylamines, N-ethylpiperidine, 2,6-lutidine, pyridine, N-methylpyrrolidine, dimethylpiperazine.

Suitable ester-forming carboxyl-blocking groups are those which may be removed under conventional conditions. Such groups for $R^x$ include methyl, ethyl, benzyl, p-methoxybenzyl, 2,4,6-trimethylbenzyl, 3,5-di-t-butyl-4-hydroxybenzyl, benzoylmethyl, p-nitrobenzyl, 4-pyridylmethyl, 2,2,2-trichloroethyl, 2,2,2-tribromoethyl, t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluenesulphonylethyl, methoxymethyl, a silyl, stannyl or phosphorus-containing group, such as described above, or an *in vivo* hydrolysable ester radical such as defined above.

The carboxyl group may be regenerated from any of the above esters by usual methods appropriate to the particular $R^x$ group, for example, acid and base catalysed hydrolysis, or by enzymically catalysed hydrolysis, or by hydrogenation.

The formation of the Schiff's base is carried out in an organic solvent or solvent mixture and, depending upon the stability of the Schiff's base, it may be isolated and purified or partially purified before reduction.

The choice of organic solvent and reaction conditions will be influenced by the solubility and reactivity of the starting materials and the resulting Schiff's base. The reaction is generally carried out in a hydrocarbon or ethereal solvent at a moderate temperature *i.e.* greater than room temperature, the reflux temperature of the solvent being selected for convenience.

The period for which the reaction is allowed to proceed depends upon the particular starting materials employed. The course of the reaction may be followed by conventional methods such as thin layer chromatography and terminated when an optimum quantity of product is present in the reaction mixture.

Suitable hydridic reducing agents for reduction of the Schiff's base include sodium borohydride and borane-trimethylamine complex. Suitable reaction solvents and reaction conditions are discussed in H.O. House "Modern Synthetic Reactions" 2nd edition chapter 2.

Suitable catalysts for use in the catalytic hydrogenation of the Schiff's base include platinum oxide and Raney nickel.

In cases where the Schiff's base is unstable or where isolation of the Schiff's base is undesirable the amino compound of formula (IV) and carbonyl compound of formula (V) may be dissolved or suspended in organic solvent, mixed, and reduced *in situ* with a hydridic reducing agent. Suitable hydridic reducing agents include sodium cyanoborohydride which is preferably used in a protic solvent such as an alcohol, in particular methanol.

The compounds of the present invention may also be prepared by reacting a compound of the formula (VI):

$$R^1\text{-CO-CO}_2R^x \qquad \text{(VI)}$$

in which $R^1$ is as defined with respect to formula (II) and $R^x$ is as defined with respect to formula (IV) with a compound of formula (VII):

$$NH_2\text{-}CH_2\text{-}R^2 \qquad \text{(VII)}$$

in which $R^2$ is as defined with respect to formula (II) followed by reduction of the adduct.

A further process for preparing the compounds of the present invention comprises the reaction of an amino compound of formula (IV) as defined hereinbefore with a compound of formula (VIII):

$$R^2\text{-X} \qquad \text{(VIII)}$$

wherein $R^2$ is as defined with respect of formula (II), and any reactive groups may be protected, and X is a halide; and thereafter where necessary carrying out steps (i) - (iii) as described hereinbefore. Preferably X is chloride, bromide or iodide.

The compounds of the present invention may also be prepared by reducing with a hydridic reducing agent an N-acyl compound of formula (IX):

$$R^2\text{-}\overset{\overset{\displaystyle O}{\|}}{C}\text{-NH-}\overset{\overset{\displaystyle R^1}{|}}{CH}\text{-CO}_2R^x \qquad \text{(IX)}$$

wherein $R^1$ and $R^2$ are as defined with respect to formula (II), $R^x$ is hydrogen or a carboxyl-blocking group, and thereafter if necessary carrying out one or more of steps (i) - (iii) as described hereinbefore.

Suitable hydridic reducing agents include the borane dimethylsulphide complex which is conveniently used in ethereal solvents such as tetrahydrofuran.

The N-acyl compounds of formula (IX) may be prepared by reacting an amino compound of formula (VI) as defined hereinbefore wherein the amino group is optionally substituted with a group which permits acylation to take place, and wherein $R^x$ is hydrogen or a carboxyl-blocking group, with an N-acylating derivative of an acid of formula (X):

$$R^2.CO_2H \qquad (X)$$

wherein $R^2$ is as defined with respect to formula (II) above.

Suitable groups which permit acylation to take place and which are optionally present on the amino group of the starting material of the formula (X) include N-silyl, N-stannyl and N-phosphorus groups, for example trialkylsilyl groups such as trimethylsilyl.

A reactive N-acylating derivative of the acid (X) is employed in the above process. The choice of reactive derivative will of course be influenced by the chemical nature of the substituents of the acid.

Suitable N-acylating derivatives include an acid halide, preferably the acid chloride or bromide. Acylation with an acid halide may be affected in the presence of an acid binding agent for example tertiary amine (such as triethylamine or dimethylamiline), an inorganic base (such as calcium carbonate or sodium bicarbonate) or an oxirane, which binds hydrogen halide liberated in the acylation reaction. The oxirane is preferably a $(C_{1-6})$-1,2-alkylene oxide , such as ethylene oxide or propylene oxide.

The acid halide may be prepared by reacting the acid (X) or a salt thereof with a halogenating (e.g. chlorinating or brominating) agent such as phosphorus pentachloride, thionyl chloride or oxalyl chloride.

Alternatively, the N-acylating derivative of the acid (X) may be symmetrical or mixed anhydride. Suitable mixed anhydrides are alkoxyformic anhydrides.

Alternative N-acylating derivatives of acid (X) are the acid azide, or activated esters such as esters with 2-mercaptopyridine, cyanomethanol, p-nitrophenol, 2,4-dimitrophenol, thiophenol, halophenols, including pentachlorophenol, monomethoxyphenol or 8-hydroxyquinoline; or amides such as N-acylsaccharins or N-acylphthalimides or an alkylidene iminoester prepared by reaction of the acid (X) with an oxime.

Other reactive N-acylating derivatives of the acid (X) include the reactive intermediate formed by reaction *in situ* with a condensing agent such as a carbodiimide, for example N,N-diethyl-, dipropyl- or diisopropylcarbodiimide, or N,N'-dicyclohexylcarbodiimide.

The condensation reaction is preferably carried out in an organic reaction medium, for example methylene chloride, dimethylformamide, acetonitrile, alcohol, benzene, dioxan, or tetrahydrofuran.

In order to put the compounds of the invention to use as medicinal agents, they are presented as pharmaceutical compositions in a variety of dosage forms.

This invention therefore also includes a pharmaceutical composition which comprises a pharmaceutically acceptable carrier or excipient together with a compound of formula (II) or a pharmaceutically acceptable salt, ester or bioprecursor thereof.

The compositions may be formulated for administration by any route, although an oral administration is preferred. The compositions may be in the form of tablets, capsules, powders, granules, lozenges, or liquid preparations, such as oral or sterile parenteral solutions or suspensions.

Unit dose presentations forms for oral administration may be tablets and capsules and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate; disintegrants, for example starch or sodium starch glycollate or pharmaceutically acceptable wetting agents such as sodium lauryl sulphate. The tablets may be coated according to methods well known in normal pharmaceutical practice. Oral liquid preparations may be in the form of, for example, aqueous or oily suspensions,

solutions, emulsions, syrups, or elixirs, or may be presented as a dry product for reconstitution with eater or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

For parenteral administration, fluid unit dosage forms are prepared utilizing the compound and a sterile vehicle, and depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, preservative and buffering agents can be dissolved in the vehicle. To enhance the stability, the composition can be frozen after filling into the vial and the water removed under vacuum. Parenteral suspensions are prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform

distribution of the compound.

The compositions may contain from 0.1% to 99% by weight, preferably from 10-60% by weight, of the active material, depending on the method of administration. The dosage employed for adult treatment will of course depend on the dose-response characteristics of the particular active ingredient but will normally be in the range 50 mg to 5 g/day.

The following Examples illustrate the preparation of the compounds of this invention.

Example 1

Preparation of N-(3,4-methylenedioxybenzyl)-L-phenylalanine

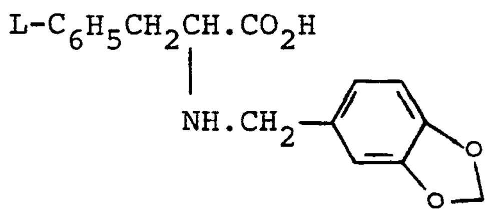

To a mixture of L-phenylalanine (3.3g, 20mM) and piperonal (3.0g, 20mM) in methanol (20ml) was added sodium cyanoborohydride (1.26g, 20mM) portionwise over ten minutes. The reaction mixture was stirred at room temperature for two hours, acidified with 10% hydrochloric acid then brought to pH 6 with 10% sodium hydroxide solution. The precipitate was filtered off, washed with water and methanol and then dried under vacuum to yield N-(3,4-methylenedioxybenzyl)-L-phenylalanine (3.7 g 62%) as a white micro-crystalline solid m.p. 226$^{o}$ (dec). (Found, C, 67.75; H, 5.97; N, 4.70% $C_{17}H_{17}NO_4$ requires C, 68.21; H, 5.73; N, 4.67% $\nu_{max}$ (KBr), 1620 $cm^{-1}$ $[\alpha]_D^{20}$ = +27.5$^{o}$ in 6N HCl/AcOH 1.1. $\delta$($d_1$-TFA) 3.5 (2H, m), 4.5 (3H, m), 6.02 (2H, s), 6.84 (3H, m), 7.18 (5H, m).

Example 2

Preparation of N-(4-chlorobenzyl)-L-phenylalanine

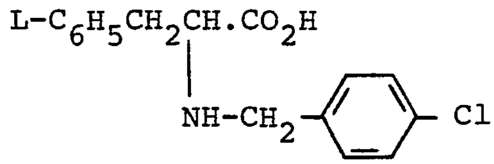

A mixture of L-phenylalanine (20g, 0.12M) and 4-chlorobenzylaldehyde (16.9g, 0.12M) in methanol (300 ml) was stirred at room temperature for 30 minutes then sodium cyanoborohydride (7.6g, 0.12M) added in one portion. The reaction mixture was stirred at room temperature overnight. The precipitate was then filtered off and washed well with methanol and water and dried under vacuum to yield N-(4-chlorobenzyl)-L-phenylalanine (20g, 58%) as a white microcrystalline solid m.p. 229-230$^{o}$ (dec) (Found, C, 65.96; H, 5.65; N, 4.77; Cl, 12.39% $C_{16}H_{16}ClNO_2$ requires C, 66.32; H, 5.56; N; 4.83; Cl, 12.24%). $\nu_{max}$ (KBr) 1620 $cm^{-1}$. $[\alpha]_D^{20}$= +30.47$^{o}$ in 6NHCl/AcOH, 1:1 $\delta$ ($d_1$-TFA), 3.45 (2H, m), 4.45 (3H m), 7.4 (9H, m) .

The compounds of Examples 3 to 10 were prepared using the method described in Example 2.

Example 3

N-(4-Chlorobenzyl)-D-phenylalanine

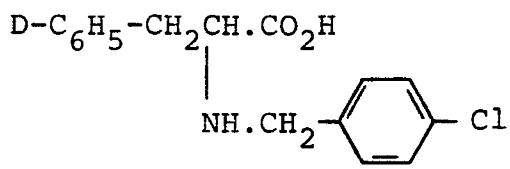

m.p. 222-224° (dec). (Found, C, 66.12; H, 5.18; N, 4.76; Cl 12.58% $C_{16}H_{16}ClNO_2$ requires C, 66.32; H, 5.56; N, 4.83; Cl, 12.34%). $\nu_{max}$ (KBr) 1620 $cm^{-1}$. $[\alpha]_D^{20} = -29.6°$ in 6N HCl/AcOH, 1:1. $\delta$ ($d_1$-TFA) 3.46 (2H, m), 4.40 (3H, m), 7.30 (9H, m).

Example 4

N-(4-Bromobenzyl)-L-phenylalanine

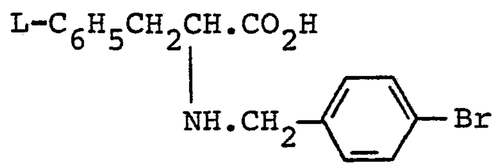

m.p. 226-228° (dec). (Found, C, 57.28; H, 4.90; N, 4.35; Br 23.60% $C_{16}H_{16}BrNO_2$ requires C, 57.49; H, 4.83; N, 4.18; (Br 23.91). $\nu_{max}$ (KBr) 1570 $cm^{-1}$. $[\alpha]_D^{20}$ = +26.0° in 6N HCl/AcOH, 1:1. $\delta$($d_1$-TFA), 3.5 (2H, m), 4.45 (3H, m), 7.3 (9H, m).

Example 5

N-(3-Trifluoromethylbenzyl)-L-phenylalanine

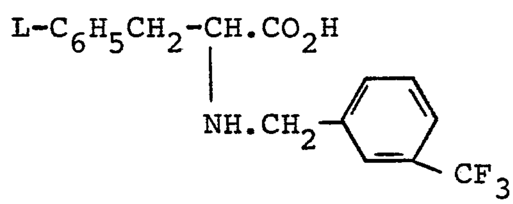

m.p. 208-210° (dec). (Found C, 63.48; H, 5.19; N, 4.33% $C_{17}H_{16}F_3NO_2$ requires C, 63.15; H, 4.99; N, 4.33%). $\nu_{max}$ (KBr) 1620 $cm^{-1}$. $[\alpha]_D^{20}$ = +33.8° in 6N HCl/AcOH 1:1. $\delta$ ($d_1$-TFA), 3.5 (2H, m) 4.5 (3H, m), 7.5 (9H, m).

Example 6

N-(4-Hydroxybenzyl)-L-phenylalanine

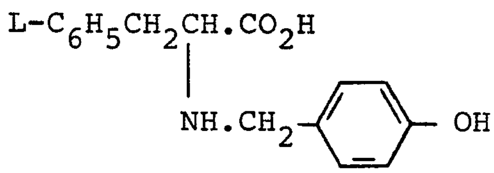

m.p. 224-226° (dec). (Found C, 71.07; H, 6.32; N, 5.11% $C_{16}H_{17}NO_3$ requires C, 70.83; H, 6.32; N, 5.16%). $\nu_{max}$ (KBr) 1780 $cm^{-1}$. $[\alpha]_D^{20}$ = +20.7° in 6N HCl/AcOH 1:1 δ(TFA) 3.5 (2H, m), 4.4 (3H, m), 7.2 (9H, m).

Example 7

N-(4-Chlorobenzyl)-DL-4-chlorophenylalanine

DL 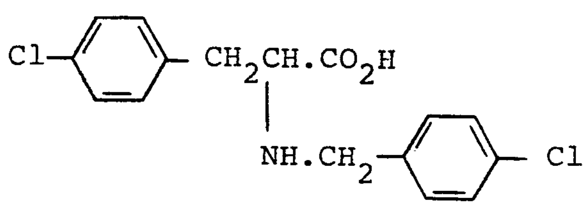

m.p. 214-217° (Found, C, 59.07; H, 4.70; N, 4.49; Cl, 21.58% $C_{16}H_{15}Cl_2NO_2$ requires C, 59.27; H, 4.66; N, 4.32; Cl, 21.87%). $\nu_{max}$ (KBr) 1605 $cm^{-1}$. δ ($d_1$-TFA), 3.43 (3H, m), 4.43 (3H, m) 7.26 (8H, 2 overlapping ABq).

Example 8

N-(4-Chlorobenzyl)-S-benzyl-L-cysteine

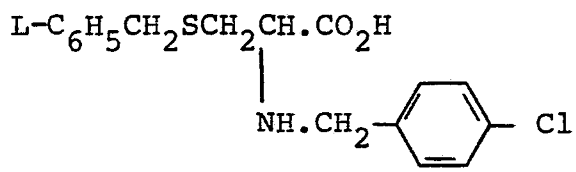

m.p. 201-202$^{\circ}$ (Found C, 59.98; H, 5.44; N, 4.22; Cl, 10.82; S, 9.48% $C_{17}H_{18}ClNO_2S$ requires C, 60.80; H, 5.40; N, 4.17; Cl, 10.56; S, 9.53%). $\nu_{max}$ (KBr) 1610 $cm^{-1}$. $[\alpha]_D^{20}$ = +21.8$^{\circ}$ in 6NHCl/AcOH 1:1. $\delta$ (TFA) 3.2 (2H m), 3.7 (2H, s), 4.0 (1H, br s), 4.2 (2H, m), 7.4 (9H, m).

Example 9

N-(4-Chlorobenzyl)-L-α-aminobenzeneacetic acid

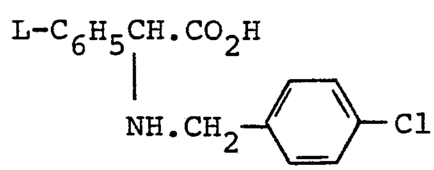

m.p. 212-213$^{\circ}$. (Found C, 64.9; H, 4.94; N, 5.08; Cl, 12.84% $C_{15}H_{14}ClNO_2$ requires C, 65.34; H, 5.08; N, 5.08; Cl, 12.87%). $\nu_{max}$ (KBr) 1565 $cm^{-1}$. $[\alpha]_D^{20}$ = +127.99 in 6NHCl/AcOH 1:1. $\delta$(TFA-$d_1$) 4.4 (2H, s), 5.25 (1H, s), 7.5 (9H, m).

Example 10

N-(4-Chlorobenzyl)-D-α-aminobenzeneacetic acid

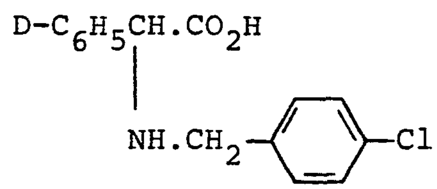

m.p. 214-215°. (Found C, 64.79; H, 5.07; N, 5.36; Cl, 13.13% $C_{15}H_{14}ClNO_2$ requires C, 65.34; H, 5.08; N, 5.08; Cl, 12.87%). $\nu_{max}$ (KBr) 1565 $cm^{-1}$. $[\alpha]_D^{20}$ = -125.82° in 6NHCl/AcOH 1:1. δ (TFA-$d_1$) 4.4 (2H, s), 5.25 (1H, s), 7.5 (9H, m).

Example 11

Preparation of DL-2-(4-Chlorobenzylamino)-3-(2-pyridyl)-propionic acid

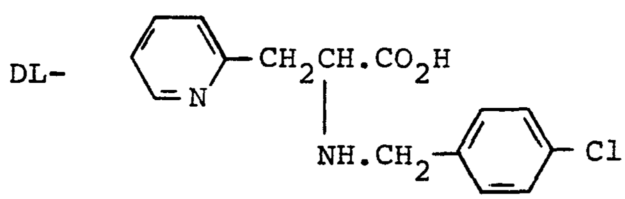

A mixture of DL-β-(2-pyridyl)-alanine dihydrobromide (4.94g, 15mM) in water (50ml) was neutralised (to pH 7) with solid sodium carbonate. Methanol (25mL), 4-chlorobenzaldehyde (2.81g, 20mM) and sodium cyanoborohydride (4.0g) 64mM were added and the mixture stirred at room temperature for 18 hours. The white precipitate which formed was filtered off, washed with water and methanol and dried to give DL-2-(4-chlorobenzylamino)-3-(2-pyridyl)-propionic acid (1.5g 34%) as a white microcrystalline solid, m.p. 214-215°.

(Found C, 61.63; H, 5.15; N, 9.27; Cl, 12.15%. $C_{15}H_{15}ClN_2O_2$ requires C, 61.96; H, 5.20; N, 9.64; Cl, 12.20%). $\nu_{max}$ (KBr) 1612 $cm^{-1}$. $\delta$($d_1$-TFA), 4.13 (2H, m), 4.63 (2H, m), 4.86 (1H, m), 7.47 (4H, S), 8.13 (2H, m) and 8.70 (2H, m).

The compound of Example 12 was also prepared using the method described in Example 11.

Example 12

DL-2-(4-Chlorobenzylamino)-3-(4-pyridyl)-propionic acid

DL -

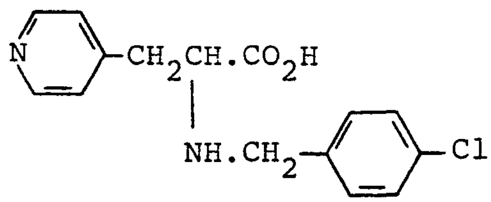

sublimes 250-253°. (Found C, 61.76; H, 5.02; N, 9.50; Cl, 12.24%. $C_{15}H_{15}ClN_2O_2$ requires C, 61.90; H, 5.20; N, 9.64; Cl, 12.20%). $\nu_{max}$ (KBr) 1615cm$^{-1}$. $\delta$($d_1$-TFA) 3.87 (2H, m), 4.56 (2H, S), 4.80 (1H, m), 7.45 (4H, S), 8.13 (2H, d, J6Hz) and 8.78 (2H, d, J6Hz).

Example 13

An alternative method of preparation of DL-2-(4-Chlorobenzylamino)-3-(2-pyridyl)-propionic acid

DL —

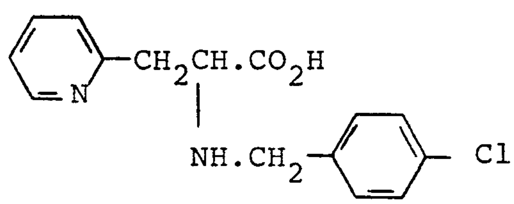

A mixture of DL-β-(2-pyridyl)alanine hydrobromide (12.3g, 37mM) in water (100ml) was neutralised (to pH 7) by stirring with excess of a prewashed ion exchange resin "Amberlite" IR-45 (OH). Filtration and evaporation of the filtrate to dryness gave the free amino acid (5.1g, 30mM), m.p. 208-210°C (Lit. m.p.

209-212$^{\circ}$C$^{+}$). The free amino acid (5.1g, 30mM) was redissolved in water (100 mL) and methanol (50mL), 4-chlorobenzaldehyde (4.5g, 30mM) and sodium cyanoborohydride (6.0g) were added. After stirring the mixture at room temperature for 18 hours the dense white precipitate was filtered off, washed with water and methanol and dried to give DL-2-(4-chlorobenzylamino)-3-(2-pyridyl)-propionic acid as a white microcrystalline solid (2.4g, 27%). Physical constants and spectral data identical to those given for Example 11.

+ G. Slater and A.W. Somerville, Tetrahedron, 23 2823 (1967).

Example 14

N-4-Chlorobenzyl-DL-tryptophan

DL-
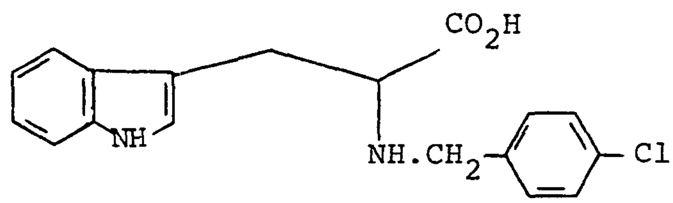

A mixture of DL-tryptophan methyl ester (4.2 g, 19mM) and 4-chlorobenzaldehyde (2.48 g, 18mM) in benzene (100 ml) was heated under reflux using a Dean and Stark apparatus for 5 minutes. The reaction mixture was cooled and evaporated to dryness to yield the crude Schiff's base, 8 g, $\nu_{max}$ (KBr) 638, 1730, 3330 $cm^{-1}$. This was hydrogenated at atmospheric pressure in methanol (100 ml) with platinum oxide (200 mg) catalyst until one equivalent of hydrogen had been absorbed. The catalyst was filtered off and the solvent evaporated to yield N-4-chlorobenzyl-DL-tryptophan methyl ester, 6 g, as a yellow oil which crystallised on standing, m.p. 96-97°, $\nu_{max}$ (KBr) 1730 $cm^{-1}$.

Hydrolysis of the methyl ester was accomplished by heating under reflux for 2 hours with aqueous methanolic sodium hydroxide (1.5 g, NaOH in 100 ml, 50% $H_2O$/MeOH). The reaction mixture was cooled, the methanol removed under reduced pressure and the residual aqueous solution diluted to 100 ml. This solution was extracted with chloroform to remove impurities, and the aqueous layer acidified with 2N hydrochloric acid. The precipitated product was filtered, washed well with water and dried under vacuum

to yield pure N-4-chlorobenzyl-DL-tryptophan (2.6 g, 45%) as a white solid, m.p. 223-228° (dec). (Found: C, 65.50; H, 5.19; N, 8.34; Cl, 10.82%; $M^+$328. $C_{18}H_{17}ClN_2O_2$ requires C, 65.75; H, 5.21; N, 8.52; Cl, 10.78%; $M^+$328). $\nu_{max}$(KBr) 1575, 1585, 3485 $cm^{-1}$. $\delta$ ($d_6$-DMSO), 3.1 (2H, m) 3.4 (1H, m), 3.88 (2H, d, J = 3Hz), 6.0 (2H, broad s, $D_2O$ exchangeable), 7.25 (9H, m) 10.82 (1H, s, $D_2O$ exchangeable).

The following compound was also prepared by the method described in Example 14.

Example 15

(DL)-2-(4-Chlorobenzylamino)-4-phenylbutyric acid

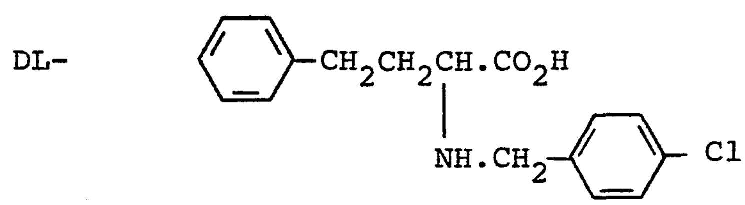

m.p. 236-237° (Found C, 67.10, H, 5.97; N, 4.82; Cl, 11.98% $C_{17}H_{18}ClNO_2$ requires C, 67.21; H 6.64; N, 4.61; Cl, 12.25%). $\nu_{max}$ (KBr) 1580 $cm^{-1}$. $\delta$ ($d_1$-TFA) 2.5 (2H, m), 2.95 (2H, m), 4.2 (3H, m), 7.25 (9H, m).

Example 16

Preparation of essentially racemised N-(4-chlorobenzyl)-α-aminobenzeneacetic acid

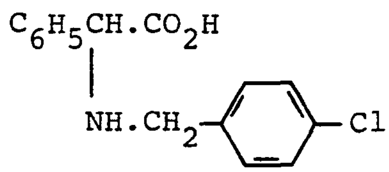

Optically pure L-α-aminobenzeneacetic acid ($[\alpha]_D^{20}$ = +154.5° in 1N HCl) was converted to the methyl ester hydrochloride by treatment with refluxing methanolic hydrogen chloride. The amino ester, liberated from the hydrochloride salt by dilute sodium bicarbonate solution, was converted to N-(4-chlorobenzyl)-α-aminobenzeneacetic acid as described in Example 14. m.p. 250° (Found, C, 64.72, H, 4.95; N, 4.98; Cl, 13.24% $C_{15}H_{14}ClNO_2$ requires C, 65.34; H, 5.08; N, 5.08; Cl, 12.87%). $\nu_{max}$ (KBr) 1585 $cm^{-1}$. $[\alpha]_D^{20}$ = +1.28° in 6N HCl/AcOH 1:1. δ($d_1$-TFA) 4.4 (2H, s), 5.28 (1H, s), 7.5 (9H, m).

Example 17

N-4-chlorobenzyl-tyrosine

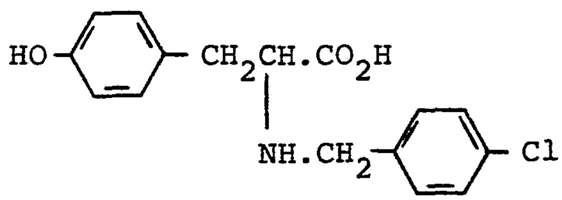

Starting from L-tyrosine and proceeding as Example 16, N-4-chlorobenzyl-tyrosine was prepared, m.p. 220-222$^oC$ (dec), $[\alpha]_D^{20}$ = 4.06$^o$ in 6NHCl/AcOH 1:1, (Found C, 62.37; H, 4.94; N, 4.44, Cl, 11.36% $C_{16}H_{16}NO_3Cl$ requires C, 62.85; H, 5.28; N, 4.58; Cl, 11.60%).

$\nu_{max}$ (KBr) 1585 $cm^{-1}$; δ (d,-TFA), 3.40 (2H,m), 4.40 (3H,m), 7.03 (4H,$A_2B_2$, J 7Hz), 7.30 (4H,$A_2B_2$, J 7Hz).

Example 18

Preparation of N-(4-chlorobenzyl)-L-phenylalanine methyl ester

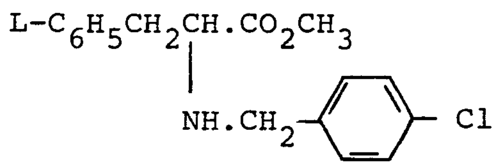

Triethylamine (3.2 ml, 2.48g, 23.18 mM) was added to L-phenylalanine methyl ester hydrochloride (5g 23.18 mmole) in ethanol (70 ml) and the mixture stirred briefly. To this solution 4-chlorobenzaldehyde (3.24g, 23.18 mM) and Adams catalyst (300 mg) was added and the mixture hydrogenated at atmospheric pressure and room temperature. After one equivalent of hydrogen had been absorbed, the reaction was stopped celite added and the mixture filtered through celite. Evaporation of the solvent yielded an oil which was partitioned between water and ether, the organic layer was washed well with water then dried (anhyd. $MgSO_4$). Removal of the drying agent and solvent yielded N-(4-chlorobenzyl)-L-phenylalanine methyl ester as an oil (5.8g 68%) (Found $M^+$ 303.1051 $C_{17}H_{18}ClNO_2$ requires 303.1026).
$\nu_{max}$(film) 1745cm$^{-1}$. $\delta$($CHCl_3$), 1.85 (1H, s), 3.0 (2H, d J=6Hz), 3.5 (1H, ABX, $J_{AX}$ = 6Hz, JAB ≃ 1Hz), 3.75 (5H, m) 7.2 (9H, m).

Example 19

N-(4-Chlorobenzyl)-L-phenylalanine methyl ester hydrochloride

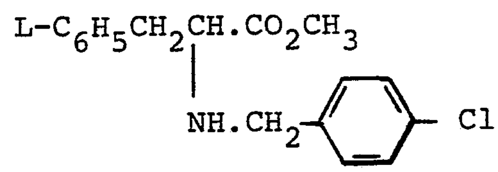

HCl

N-(4-Chlorobenzyl)-L-phenylalanine methyl ester (1.1 g) from previous example in dry ether (20 ml) was treated with dry ether saturated with hydrogen chloride until no further precipitate formed. The solid was filtered off and recrystallised from isopropyl alcohol/dry diethyl ether to yield N-(4-chlorobenzyl)-L-phenylalanine methyl ester hydrochloride as white needles (0.82 g, 66%) m.p. 148-150$^{o}$ (Found C, 60.12; H, 5.28; N, 4.11; Cl, 20.82% $C_{17}H_{19}Cl_2N_2O_2$ requires C, 60.00; H, 5.63; N, 4.12 Cl; 20.84%). $\nu_{max}$ (KBr) 1750 $cm^{-1}$. $[\alpha]_D^{20}$ = +32.94$^{o}$ in 6NHCl/AcOH, 1:1) δ ($CDCl_3$), 3.6 (6H, singlet on a complex multiplet) 4.18 (2H, s), 7.4 (9H, m).

For each of the above examples, the other optical forms of the compounds (L-, D- or DL-) may be prepared using an appropriate optical form (L-, D- or DL-) of amino acid reactant. Thus, N-(4-chlorobenzyl)-D-phenylalanine may be prepared by using D-phenylalamine in place of L-phenylalanine in Example 2. Similarly, L- or D-2-(4-chlorobenzylamino)-3-(2-pyridyl)-propionic acid may be prepared by using D- or L-β-(2-pyridyl)-alanine in place of DL-β-(2-pyridyl)-alanine in Example 11.

## Biological Data

The compounds of the Examples above were tested for their effects on serum lipids *in vivo* using normolipidaemic rats.

## Protocol

Male Sprague-Dawley rats, 80-100 g, were randomised into groups of 8 after 5-7 days of feeding on the diet. The compounds were fed to the rats at a concentration of 0.1% of the powered diet + (i.e. approximately 100 mg/kg/day), unless otherwise stated. The control groups were fed only the diet. At the end of the experiment, i.e. after 7 days, the rats were killed and their individual total serum cholesterol and triglyceride measured in mg/100 ml using a Technicon Autoanalyser. The high density lipoprotein (HDL)-cholesterol fraction, which was obtained from the serum by precipitation with heparin-manganous chloride, was only measured on "pooled" samples from each group.

The results are shown in Table 1 below.

+ Model A used an Oxoid diet.
Model B used a semi-synthetic diet rich in sucrose and cholesterol

TABLE 1

| Compound of Example No. | Rat Model | Percentage difference≠ from control values in | | |
|---|---|---|---|---|
| | | Total Serum cholesterol | Total Serum triglyceride | High Density lipo-protein (HDL) cholesterol° |
| 1 | A | + 8 | -31* | + 10 |
| 2 | A | +31* | -40* | + 20 |
| | B | +14 | -44* | + 24 |
| 3 | B | +49* | -67* | + 68 |
| 4 | A | +43* | -40* | + 40 |
| 5 | A | +47* | -41* | + 51 |
| 6 | A | + 9 | - 3 | + 14 |
| 7 | A | +56* | -50* | + 66 |
| 8 | A | +52* | -35* | + 61 |
| 11 | B | +27* | +27 | + 43 |
| 12 | B | -10 | -10 | + 14 |
| 14 | A | +36* | - 8 | + 29 |
| 15 | A | +68* | -11 | + 62 |
| 16+ | A | +79* | + 7 | +104 |
| 17 | A | - 9 | -18 | + 18 |
| 18 | A | +25* | -43* | + 56 |

\+ This compound was tested at 0.05% of the diet

° Analysis carried out on pooled samples

≠ (-) indicates lower and (+) indicates higher than control values

* Statistically significant at $p<0.05$

CLAIMS:

1. A compound of the formula (II) or a pharmaceutically acceptable salt, ester or bioprecursor thereof:

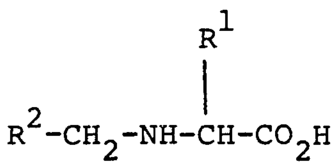

(II)

wherein $R^1$ represents an optionally substituted aryl, aralkyl, aromatic heterocyclic or aromatic heterocyclylalkyl group, and $R^2$ represents a phenyl group optionally substituted with $C_{1-6}$ alkyl, halogen, hydroxy, trifluoromethyl or methylenedioxy, with the proviso that when $R^1$ is benzyl, $R^2$ is phenyl, 4-chlorophenyl, 2-hydroxyphenyl, $\alpha$-$C_{10}H_7$- or $\beta$-$C_{10}H_7$-, the compound of formula (II) is not in the DL-form.

2. A compound according to claim 1, in which $R^1$ represents phenyl or benzyl optionally substituted with halogen, lower alkyl or hydroxyl, or a 5 or 6 membered heterocyclic group containing one or more nitrogen, oxygen or sulphur atoms and optionally substituted with lower alkylene, or a bicyclic aromatic heterocyclic group containing one or more nitrogen, oxygen or sulphur atoms.

3. A compound according to claim 1 or claim 2, in which $R^2$ is a 4-halophenyl group.

4. A compound according to any one of claims 1 to 3, in which $R^1$ is an indolylmethyl or 2-picolyl group.

5. A compound according to claim 1, of general formula (III) or a pharmaceutically acceptable salt, ester or bioprecursor thereof.

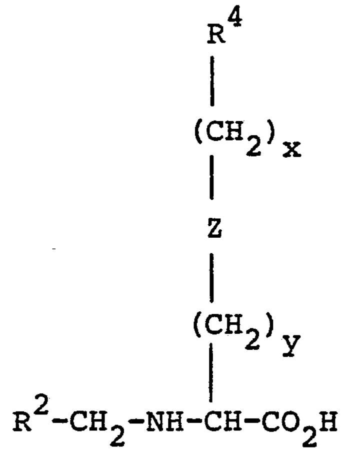

(III)

wherein $R^2$ is as defined with respect to formula (II), $R^4$ is phenyl optionally substituted with halogen or hydroxy, a 5 or 6 membered heterocyclic group containing one or more nitrogen, oxygen or sulphur atoms, or a bicyclic aromatic heterocyclic group containing one or more nitrogen, oxygen or sulphur atoms, x and y are each an integer of from 1 to 4, and Z is an oxygen or sulphur atom or is a bond.

6. A compound according to claim 5, in which $R^4$ is a phenyl, 4-hydroxyphenyl, 4-chlorophenyl, indolyl or pyridyl group.

7. A compound according to claim 5 or 6, in which x and y are each 1 or 2.

8. A compound according to any one of claims 1 to 7, in the optically active L-form.

9. A process for preparing a compound of formula (II) which comprises reacting an amino compound of formula (IV):

$$\begin{array}{c} R^1 \\ | \\ H_2N\text{-}CH\text{-}CO_2R^x \end{array} \qquad (IV)$$

wherein $R^1$ is as defined with respect to formula (II) and any reactive groups therein may be protected, and $R^x$ is hydrogen or a carboxy blocking group, with a carbonyl compound of formula (V):

$$R^2\text{-}CHO \qquad (V)$$

wherein $R^2$ is as defined with respect to formula (II) and any reactive groups may be protected, followed by reduction of the resulting Schiff's base with a suitable hydridic reducing agent, or hydrogen in the presence of a catalyst; and thereafter, if necessary, carrying out one or more of the following steps:

i) removing any carboxyl-blocking group $R^x$;
ii) removing any protecting groups on $R^1$ or $R^2$;
iii) converting the product into a pharmaceutically acceptable salt, ester or a pharmaceutically acceptable bioprecursor.

10. A process for preparing a compound of formula (II), which comprises reacting a compound of the formula (VI):

$$R^1-CO-CO_2R^x \qquad (VI)$$

in which $R^1$ is as defined with respect to formula (II) and $R^x$ is as defined with respect to formula (IV) with a compound of formula (VII):

$$NH_2-CH_2-R^2 \qquad (VII)$$

in which $R^2$ is as defined with respect to formula (II), followed by reduction of the adduct.

11. A process for preparing a compound of formula (II), which comprises reacting an amino compound of formula (IV) as defined in claim 9 with a compound of formula (VIII):

$$R^2-X \qquad (VIII)$$

wherein $R^2$ is as defined with respect to formula (II), and any reactive groups may be protected,

and X is a halide; and thereafter where necessary carrying out steps (i) - (iii) as described in claim 9.

12. A process for preparing a compound of formula (II) which comprises reducing with a hydridic reducing agent an N-acyl compound of formula(IX):

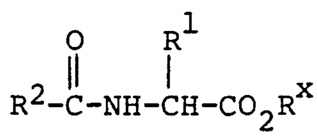

(IX)

wherein $R^1$ and $R^2$ are as defined with respect to formula (II), $R^x$ is hydrogen or a carboxyl-blocking group, and thereafter, if necessary, carrying out one or more of steps (i) - (iii) as described in claim 9.

13. A pharmaceutical composition comprising a compound according to any one of claims 1 to 8, or when made by a process according to any one of claims 9 to 12 together with a pharmaceutically acceptable carrier or excipient.

14. A pharmaceutical composition according to claim 13, in unit dosage form.

CLAIMS: FOR AUSTRIA

1. A process for preparing a compound of formula (II) or a pharmaceutically acceptable salt, ester or bioprecursor thereof:

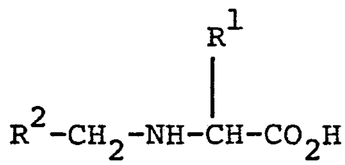

(II)

wherein $R^1$ represents an optionally substituted aryl, aralkyl, aromatic heterocyclic or aromatic heterocyclylalkyl group, and $R^2$ represents a phenyl group optionally substituted with $C_{1-6}$ alkyl, halogen, hydroxy, trifluoromethyl or methylenedioxy, with the proviso that when $R^1$ is benzyl, $R^2$ is phenyl, 4-chlorophenyl, 2-hydroxyphenyl, $\alpha$-$C_{10}H_7$- or $\beta$-$C_{10}H_7$-, the compound of formula (II) is not in the DL-form, which comprises reacting an amino compound of formula (IV):

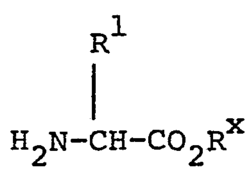

(IX)

wherein $R^1$ is as defined with respect to formula (II) and any reactive groups therein may be protected, and $R^x$ is hydrogen or a carboxy blocking group, with a carbonyl compound of formula (V):

$$R^2\text{-CHO} \qquad (V)$$

wherein $R^2$ is as defined with respect to formula (II) and any reactive groups may be protected, followed by reduction of the resulting Schiff's base with a suitable hydridic reducing agent, or hydrogen in the presence of a catalyst; and thereafter, if necessary, carrying out one or more of the following steps:

i) removing any carboxyl-blocking group $R^x$;
ii) removing any protecting groups on $R^1$ or $R^2$;
iii) converting the product into a pharmaceutically acceptable salt, ester or a pharmaceutically acceptable bioprecursor.

2. A process for preparing a compound of formula (II) defined in claim 1, which comprises reacting a compound of the formula (VI):

$$R^1\text{-CO-CO}_2R^x \qquad (VI)$$

in which $R^1$ is as defined with respect to formula (II) and $R^x$ is as defined with respect to formula (IV), with a compound of formula (VII):

$$NH_2\text{-CH}_2\text{-R}^2 \qquad (VII)$$

in which $R^2$ is as defined with respect to formula (II), followed by reduction of the adduct.

3. A process for preparing a compound of formula (II) as defined in claim 1, which comprises reacting an amino compound of formula (IV) as defined in claim 1 with a compound of formula (VIII):

$$R^2\text{-}X \qquad \text{(VIII)}$$

wherein $R^2$ is as defined with respect to formula (II), and any reactive groups may be protected, and X is a halide; and thereafter where necessary carrying out steps (i) - (iii) as described in claim 1.

4. A process for preparing a compound of formula (II) as defined in claim 1, which comprises reducing with a hydridic reducing agent an N-acyl compound of formula (IX):

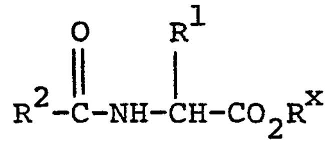

(IX)

wherein $R^1$ and $R^2$ are as defined with respect to formula (II), $R^x$ is hydrogen or a carboxyl-blocking group, and thereafter, if necessary, carrying out one or more of steps (i) - (iii) as described in claim 1.

5. A process according to any one of claims 1 to 4, in which $R^1$ represents phenyl or benzyl optionally substituted with halogen, lower alkyl or hydroxyl, or a 5 or 6 membered heterocyclic group containing one or more nitrogen, oxygen or sulphur atoms and optionally substituted with lower alkylene, or a bicyclic aromatic heterocyclic group containing one or more nitrogen, oxygen or sulphur atoms.

6. A process according to any one of claims 1 to 5, in which $R^2$ is a 4-halophenyl group.

7. A process according to any one of claims 1 to 6, in which $R^1$ is an indolylmethyl or 2-picolyl group.

8. A process according to any one of claims 1 to 7, in which the compound of formula (II) is in the optically active L-form.

0031653

Application number

EP 80 30 4407

European Patent Office

# PARTIAL EUROPEAN SEARCH REPORT

which under Rule 45 of the European Patent Convention shall be considered, for the purposes of subsequent proceedings, as the European search report

## DOCUMENTS CONSIDERED TO BE RELEVANT

| Category | Citation of document with indication, where appropriate, of relevant passages | Relevant to claim |
|---|---|---|
| X | US - A - 3 850 968 (S. TOYOSHIMA et al.)<br>* Column 1, line 37 - column 2, line 29; example 5 *<br>-- | 1,2,<br>8-14 |
| X | CHEMICAL ABSTRACTS, vol. 68, no. 11, March 11, 1968, page 4862, abstract 50017k Columbus, Ohio, USA<br>A.N. KOST et al. "α-Arylmethylamino acids".<br>& KHIM. FARM. ZH. 1(2) 3-7 (1967)<br>* Whole abstract *<br>-- | 1,2,4<br>8-12 |
| X | BULLETIN OF THE CHEMICAL SOCIETY OF JAPAN,vol.46, no. 1, 1973,<br>Tokyo, JP<br>M.MIYOSHI: "Peptide synthesis via N-acylated aziridinone. I. The synthesis of 3-substituted-1-benzyloxycarbonylaziridin-2-ones and related compounds", pages 212-218.<br>./. | 1,2,8-<br>12 |

**CLASSIFICATION OF THE APPLICATION (Int. Cl.³)**

C 07 C 101/04
C 07 D 209/20
C 07 D 213/55
C 07 D 317/38
C 07 C 149/24
A 61 K 31/195
31/445
31/405
31/34

**TECHNICAL FIELDS SEARCHED (Int. Cl.³)**

C 07 C 101/04
C 07 D 209/20
C 07 D 213/55
A 61 K 31/195
31/445
31/405

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:

Claims searched incompletely: 1-8

Claims not searched:

Reason for the limitation of the search:

The expression "bioprecursor" (claims 1,5) is not sufficiently clear, it has not been taken into consideration during the search.

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| Place of search | Date of completion of the search | Examiner |
|---|---|---|
| The Hague | 24-03-1981 | PAUWELS |

European Patent Office

# PARTIAL EUROPEAN SEARCH REPORT

Application number EP 80 30 4407



| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| | * Page 218, lines 2-5 *<br>-- | | |
| X | CHEMICAL ABSTRACTS, vol. 73, no. 5, August 3, 1970, page 325, abstract 25030e Columbus, Ohio, USA DAVTYAN S.M. et al. "Derivatives of γ-aminobutyric acid. I. Synthesis of methyl esters of N-substituted α-phenylaminoacetic acids and products of their reduction"<br>& ARM.KHIM. ZH. 1970, 23(4), 251-7<br>* Whole abstract *<br>-- | 1,2,8-12 | |
| | | | TECHNICAL FIELDS SEARCHED (Int. Cl.3) |
| X | CHEMICAL ABSTRACTS, vol. 45 no. 18, September 25, 1951, abstract 7957 Columbus, Ohio, USA SEIZO KANAO: "Alkylamino acids".<br>& J. PHARM SOC. JAPAN 66, II. Ibid 6-7<br>* Whole document *<br>-- | 1,2,4, 8-12 | |
| X | FR - A - 1 100 016 (U.C.L.A.F.)<br>* Page 3, column 1, example 6 *<br>-- | 1,2,4, 8-12 | |
| X | US - A - 3 950 542 (G. KALOPISSIS)<br>* Column 1, line 19 - column 3, line 27 *<br>---- | 1,5, 8-14 | |